# EUROPEAN PATENT SPECIFICATION

(11) **EP 0 382 158 B1**
(45) Date of publication and mention of the grant of the patent: **13.04.1994**
(21) Application number: 90102295.4
(22) Date of filing: 06.02.1990
(51) Int. Cl.: A61F 2/06, A61L 27/00

(54) **Prosthesis for living organ**
Prothese für ein lebendes Organ
Prothèse pour organe vivant

(30) Priority: 07.02.1989 JP 27812/89
(43) Date of publication of application: 16.08.1990
(73) Proprietor: TERUMO KABUSHIKI KAISHA, Tokyo 151 (JP)
(72) Inventor: Noishiki,Yasuharu, Tohaku-gun, Tottori-ken (JP); Matsumoto, Atsushi, c/o Terumo Kabushiki Kaisha, Ashigarakami-gun, Kanagawa-ken (JP); Igawa, Satoshi, c/o Terumo Kabushiki Kaisha, Ashigarakami-gun, Kanagawa-ken (JP); Nishihara, Katsunori, c/o Terumo Kabushiki Kaisha, Ashigarakami-gun, Kanagawa-ken (JP); Okumura, Tadashi, c/o Terumo Kabushiki Kaisha, Ashigarakami-gun, Kanagawa-ken (JP); Katakura, Takeo, c/o Terumo Kabushiki Kaisha, Ashigarakami-gun, Kanagawa-ken (JP)
(74) Representative: Casalonga, Axel

(56) References cited:
- EP-A- 0 194 192
- WO-A-80/02641
- FR-A- 2 391 709
- FR-A- 2 541 888
- US-A- 4 693 720

## Description

### BACKGROUND OF THE INVENTION

This invention is directed to prostheses for living organ adapted for such use as artificial blood vessel, heart, and patch for blood vessel. This invention is also directed to artificial blood vessels prepared from such prosthesis and a process for preparing thereof.

Various artificial blood vessels have been developed and are now on the market. Artificial blood vessels and prostheses (patches) fabricated from fabrics woven of polyesters and the like are the typical examples.

Since internal and external areas of the commonly used porous artificial blood vessels fabricated from fabrics woven of polyesters and the like are communicable through the wall, tissues around an implanted artificial blood vessel invade the vessel and form a stable pseudo-endothelium, thus enabling the vessel to be patent stably for a long period. Therefore, quick and stable formation of the pseudo-endothelium which has a natural anti-thrombotic property is an important factor for the healing of the implanted artificial blood vessel, and, for this purpose, penetration of tissues into the porous layer of the artificial blood vessel should be achieved as quickly as possible.

In addition, permeable porous materials, such as polyester fabrics, are being applied to commonly used artificial blood vessels, in order to ensure the penetration ability of tissues after implantation. However, the use of such a kind of artificial blood vessel requires complicated handling, because it is essential to perform a pre-clotting treatment (a treatment to clot the blood vessel wall with a patient's own blood) in order to repress bleeding at the time of an implantation operation. Even after an implantation operation, the use of such a kind of artificial blood vessel is still attended with a danger of causing bleeding due to fibrinolysis (melting of the pretreated clots).

WO 80/02641 discloses a blood vessel prosthesis comprising an outer tube of a porous essentially non-resorbable material, and an inner covering or tube of a resorbable material in the form of a net or a porous tube structure. To obtain the endothelium layer, the resorbable inner wall material should have such a porous structure that blood cells, such as erythrocytes and thrombobcytes, may pass into or through it.

In view of the above, it therefore becomes an object of the present invention to provide a prosthesis for living organ to be implanted in a body, which renders possible (1) reduction of the risk of causing contamination by pieces of thread or fraying at a cut site of the prosthesis at the time of implantation operation, (2) prevention of bleeding from the wall of an artificial blood vessel after its implantation, and (3) enhancement of early stage penetration of surrounding tissues into the artificial blood vessel by the effect of the prosthesis to prevent outward leakage of blood plasma from the blood-flowing side and further penetration of the tissues by which the exterior of the vessel get into communication with the blood-flowing side after decomposition and absorption of the inner layer of the wall.
In accordance with the present invention, there is provided a prosthesis for living organ to be implanted in a body at such site wherein one major surface of the prosthesis contacts with blood, comprising a laminate of
a first layer comprising an absorbable material which is capable of being gradually absorbed through decomposition or metabolism of the material such that the absorbable material is removed from the prosthesis within the survival period of the patient,
said first layer being provided on the blood-contacting side of the prosthesis, and
a water-permeable second layer primarily comprising a non-absorbable material for supporting the prosthesis and imparting strength thereto, which material is not absorbed in the body within the lifetime, characterized in that said first layer is substantially water-impermeable, and said prosthesis becomes water-permeable after removal of said absorbable material.

Preferably, said non-absorbable material is in a form of a porous structure and said absorbable material is a biocompatible material containing an anti-thrombotic material. Preferably, said substantially water-impermeable first layer is in a form of a porous structure at least on its blood-contacting side. Preferably, said absorbable material is an absorbable polyester selected from the group consisting essentially of polylactate, polyglycolate, polyhydroxybutylate, polyhydroxyvalerate, poly-ε-caprolactone ester, polyethylene adipate and their copolymers.

Preferably, said absorbable polyester contains at least a member selected from the group consisting essentially of polyamino acids, phospholipids and fatty acids. Preferably, said non-absorbable material is selected from the group consisting essentially of polyesters, polyurethanes, polyethylenes, polypropylenes, fluoroplastics, and Teflon. Preferably, said porous structure comprises a fabric knitted or woven of respective material. Also preferably, said first layer comprises said water-impermeable material impregnated in a water-permeable material.

In addition, in accordance with the present invention, there is provided the process of preparing the prosthesis for living organ to be implanted in a body, as described above, wherein a water-impermeable first layer comprising an absorbable material is formed on a water-permeable second layer primarily containing a non-absorbable material by inserting a cylindrical form of film comprising said absorbable material into at least a portion of cavity of an artificial blood vessel prepared from said second layer.

The above-described prosthesis for living organ to be implanted in a body can be applied to an artificial blood vessel.

Other objects and advantages of the present invention will be made apparent as the description progresses.

### BRIEF DESCRIPTION OF THE DRAWINGS

Fig. 1 is a cross sectional view of a prosthesis for living organ to be implanted in a body, showing one embodiment of the present invention.

Fig. 2 is a cross sectional view of a prosthesis for living organ to be implanted in a body, showing another embodiment of the present invention.

### DETAILED DESCRIPTION OF THE INVENTION

The present inventors have conducted intensive studies on a prosthesis for living organ to be implanted in a body based on an assumption that permeation of liquid components in blood from the interior of an artificial blood vessel to its external area and dispersion of a wound-flud formed in a porous structure of the vessel into blood could be the cause of the delayed penetration of surrounding tissues into the vessel, and have accomplished the present invention as a result of these efforts.

In the present situation, such a penetration of surrounding tissues after implantation of a commonly used artificial blood vessel makes slow progress, because a pre-clotting treatment (a treatment to fill up porous structure of artificial blood vessel with thrombus) which is performed at the time of implantation of the vessel can not prevent permeation of liquid components in blood and dispersion of a wound-flud into blood, even if this treatment can prevent substantial leakage of blood.

On the contrary, penetration of surrounding tissues into an artificial blood vessel made of a prosthesis of the present invention progresses efficiently without receiving any inhibition by liquid components in blood, because a water-impermeable layer at least a part of it containing an absorbable material is formed on the inside surface of a water-permeable porous structure made of a non-absorbable material and such a inner layer prevents passage of liquid components in blood through the wall of the artificial blood vessel at the time of its implantation. The water-impermeable layer in the artificial blood vessel described above is then decomposed and absorbed after the implantation and, thereafter, the surrounding tissues start to penetrate further inside. Since capillary blood vessels are already formed sufficiently in the penetrated tissues at the time of this second penetration, endothelial cells contained in the capillary blood vessels reach inner wall of the artificial blood vessel and form pseudo-endothelium smoothly.

The following describes the prosthesis for living organ to be implanted in a body in accordance with the present invention in detail.

Among artificial blood vessels which are now on the market, any of the porous blood vessels fabricated from polyester fabrics and the like fulfils its function as an active blood vessel after the penetration of surrounding tissues into its porous structure, which renders possible stable adhesion of new pseudo-endothelium and stable operability for a long period. Such a penetration of tissues is a kind of the expression of foreign body recognition, which occurs against not only a implanted artificial blood vessel but also, more or less, any other porous structure implanted in the body. In the case of the implantation of an artificial blood vessel, damaged natural blood vessels at the anastomotic part obviously undergo an influence of the change of wound-healing function, but such an influence lessens as the distance from the anastomotic part lengthens and, therefore, surrounding tissues recognize the artificial blood vessel as an implanted foreign body.

In consequence, an experiment was performed by using a set of artificial blood vessels which have previously been subjected to a pre-clotting treatment, in order to compare the penetration ability of body tissues after the implantation of the vessel into subcutaneous part of thorax or into thoracic cavity with the penetration ability after its implantation to thoracic aorta. Results of the comparative experiment are shown in Table 1 in terms of the elapsed time (week) after the implantation.

**Table 1**

| Time lapse (week) | Thorax (subcutaneous) | Thoracic cavity | Thoracic aorta | |
|---|---|---|---|---|
| | | | blood side | cavity side |
| 1 | + | + | - | + |
| 2 | ++ | ++ | + | + |
| 4 | +++ | +++ | + | ++ |
| Symbols -, +, ++ and +++ indicate increasing degree of the intrusion of tissues into the artificial blood vessel structure in that order. | | | | |

It was found from Table 1 that the penetration of tissues in the case of the subcutaneous implantation or implantation into thoracic cavity obviously starts at earlier stage than in the case of the implantation into thoracic aorta and, in the latter case, the penetration of tissues into the thoracic cavity side starts early compared to that into the blood flow side.

Based on an assumption that such a delay in the penetration of tissues into the implanted artificial blood vessel is originated from a result of the repression of inflammation by blood plasma leaked out from the blood flow side, inner surface of an artificial blood vessel made of a polyester fabric was coated with a silicone film to prevent leakage of blood plasma and the coated vessel was implanted into the thoracic aorta. As the result, remarkable tissue penetration enhancing effect was observed. In addition, development of many capillary blood vessels for nutrient supply was observed in tissues just below the implanted artificial blood vessel.

In general, healing of artificial blood vessel is completed by the formation of stable endothelium (endothelial cell layer, smooth muscle cell layer) and exothelium (loose connective tissue layer). In practice, however, quick formation of stable endothelium is the most important subject to be solved.

Though there are many items which remain still unexplained with regard to the mechanism of endothelium formation in an artificial blood vessel, the following two factors seem to be the main cause of the endothelium formation: 1), creeping of the existing endothelium from inside of the natural blood vessel to be connected into the implanted artificial blood vessel at the anastomotic site and 2), participation of capillary blood vessel-originated endothelial cells in the endothelium formation as a result of the penetration and opening of nutritive blood vessels formed in the exothelial layer into the blood flow area inside the artificial blood vessel.

Therefore, it is advisable that an exothelial layer rich in nutritive blood vessels be formed quickly for the purpose of forming a stable endothelium at an early stage, and the curing-enhancing effect of a water-impermeable layer coated on the inner surface of an artificial blood vessel has a great meaning to achieve such a purpose.

Consequently, in accordance with the present invention, there is provided a prosthesis for living organ to be implanted in a body at such site wherein one major surface of the prosthesis contacts with blood, comprising a laminate of
a first layer of an absorbable material which is capable of being gradually absorbed through decomposition or metabolism of the material such that the absorbable material is removed from the prosthesis within the survival period of the patient,
said first layer being provided on the blood-contacting side of the prosthesis, and
a water-permeable second layer primarily comprising a non-absorbable material for supporting the prosthesis and imparting strength thereto, which material is not absorbed in the body within the lifetime, whereas said first layer is substantially water-impermeable, and said prosthesis becomes water-permeable after removal of said absorbable material.

The prosthesis for living organ to be implanted in a body according to the present invention is a material which is used to patch up a blood-contacting area and is applicable to a tubular artificial blood vessel, a sheet of patch and the like.

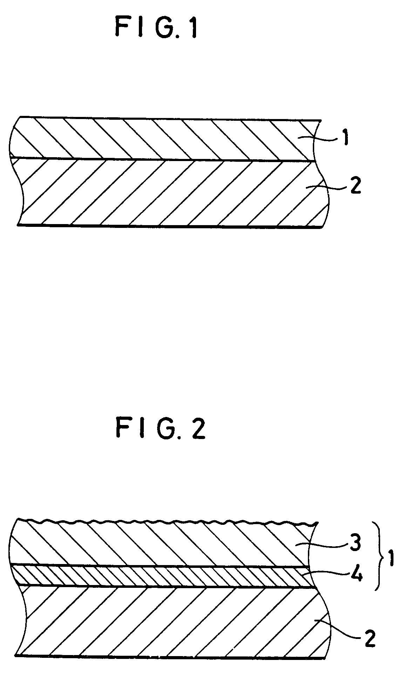

Fig. 1 is a cross sectional view of a prosthesis for living organ to be implanted in a body, showing an appropriate embodiment of the present invention. The prosthesis of the present invention comprises at least a water-impermeable first layer 1 and a water-permeable second layer 2.

The water-impermeable first layer contains an absorbable material which is capable of being gradually absorbed through decomposition or metabolism of the material such that the absorbable material is removed from the prosthesis within the survival period of the patient, the water-impermeable layer 1 of the prosthesis consequently becoming water-permeable.

Fig. 2 is a cross sectional view of a prosthesis for living organ to be implanted in a body, showing another appropriate embodiment of the present invention. In the case of the example shown in Fig. 2, the water-impermeable first layer 1 consists essentially of an absorbable layer 3 comprising an absorbable material wherein its surface is in a form of porous structure and a supporting layer 4 which comprises a water-permeable non-absorbable material. This first layer comprising these two sublayers is formed on a water-permeable second layer 2. The water-impermeable first layer 1 consisting of the absorbable layer 3 and the supporting layer 4 may comprise either two layers as shown in Fig. 2 or a single layer in which the absorbable layer 3 and the supporting layer 4 are laminated by, for example, impregnating an absorbable material into a supporting layer 4 comprising a knitted or woven fabric.

The substantially water-impermeable first layer may be composed of either an absorbable material as the sole material or a complex of an absorbable material and a porous structure made of a non-absorbable material. The water-permeable second layer is in a form of a water-permeable porous structure which may be composed of either a porous structure made solely of a non-absorbable material or a complex of an absorbable material and a porous structure made of a non-absorbable material.

The term "absorbable material" as used herein and in the claims refers to a material which is capable of being gradually absorbed through decomposition or metabolism of the material such that the absorbable material is vanished from the prosthesis within the survival period of the patient, and, in the same manner, the term "non-absorbable material" refers to a material which can maintain its strength and structure without being absorbed in the body of the patient within the lifetime.

The term "absorbable" as used herein and in the claims is intended to mean decomposition absorption and/or metabolic absorption abilities, more specifically, the decomposition absorption means natural decomposition of a material into smaller molecules and subsequent absorption and the metabolic absorption means metabolic decomposition of a material into smaller molecules and subsequent absorption.

With regard to the terms "water-permeable" and "water-impermeable" as used herein and in the claims, the term "water-impermeable" means that a material has a coefficient of water permeability of 0 ml/cm²·min and the term "water-permeable" means that a material has a coefficient of water permeability of other than 0 ml/cm²·min, provided that the coefficient of water permeability is defined as the outflow of water per minute per 1 cm² area of an artificial blood vessel or a material for use in blood vessel patching under a water pressure of 16 kPa (20 mmHg). In the practice of the present invention, however, "substantially water-impermeable" may be sufficient enough to satisfy the meaning of the term "water-impermeable", and a "substantially water-impermeable material" may include a material which has a permeability in some degree but within a range that does not alter the effect of the present invention.

In the case of commonly used artificial blood vessels having a porous structure made of polyester fabrics and the like, it is essential to perform a complicated pre-clotting treatment using a patient's own blood in order to prevent bleeding at the time of implantation, in addition to a danger of causing bleeding due to the enhancement of fibrinolysis even after the implantation operation. These problems have a mutual relation to the post-implantation healing, with a contradictory fact that penetration of tissues into an artificial blood vessel and the post-implantation healing are achieved efficiently when the vessel has higher permeability and higher frequency of causing bleeding due to fibrinolysis and the like. Contrary to these prior art problems, the prosthesis for living organ to be implanted in a body in accordance with the present invention does not require a pre-clotting treatment and can prevent bleeding after implantation operation because of the application of the absorbable and water-impermeable layer, as well as its capacity for increasing coefficient of water permeability of the non-absorbable porous structure which renders possible improvement of the penetration ability of tissues.

It should be noted that the absorbable material for use in the water-impermeable layer must be a substance which has no toxicity even after its decomposition and absorption in the body and does not debase significantly any of the physical properties that are required for artificial blood vessels, such as flexibility and adaptabilities to needle-injection and suture. In addition to the above-described pre-clotting treatment making use of a patient's own blood, several pre-treatments are being performed using albumin, fibrin, collagen, gelatin and the like, for the purpose of preventing leakage of blood from inside of the commonly used porous artificial blood vessel. However, all of these substances have hydrophilic properties and, therefore, absorb water easily. Moreover, the use of some of them, for example fibrin and collagen, can prevent bleeding but can not prevent leakage of blood plasma because of their fibrous net structures.

Therefore, the absorbable material for use in the prosthesis of the present invention may preferably be a substance which hardly contains water, has no toxicity even after its decomposition in the body and shows practically no foreign body reaction, such as absorbable polyesters including polylactate, polyglycolate, polyhydroxybutylate, polyhydroxyvalerate, poly-ε-caprolactone ester, polyethylene adipate and copolymers thereof or a mixture of polyesters having water-absorbing ability, though these substances are in no way to be taken as limiting.

Communication between inner and outer areas of the prosthesis of the present invention should be started after penetration of surrounding tissues into the water-impermeable layer at least to an extent that can prevent bleeding and subsequent decomposition and absorption of the absorbable material in the water-impermeable layer. Since the starting time of such a communication is affected by the implanting part of an artificial blood vessel, diameter of the vessel and the like, decomposition rate and related properties are controlled by selecting a proper absorbable material or mixing various absorbable materials.

For example, the decomposition rate can be controlled within a range from several days to several months by using a mixture of a copolymer of polylactate and polyglycolate and a copolymer of polyhydroxybutylate and polyhydroxyvalerate, because the former copolymer is decomposed and absorbed within several days to several weeks while the latter copolymer is decomposed and absorbed within several months. In addition, because these absorbable polyesters crystallize at room temperature, changing of their crystallinities by mixing them with phospholipids, fatty acids and the like renders possible control of the rate of decomposition and absorption and improvement of needle-injection capability, flexibility and the like, which are necessary physical properties for artificial blood vessel. These regulation and improvement may also be achieved by mixing an absorbable polyester with an absorbable material which has been used in the art, such as gelatin, albumin, fibrin, collagen and polyleucine.

In the case of an artificial blood vessel having a small diameter, an absorbable material may preferably contain an anti-thrombotic agent, such as heparin, in order to prevent obstruction caused by excess formation of thrombi during a period between the exothelium formation and decomposition and absorption of the absorbable material.

In general, an artificial blood vessel is cut just prior to its implantation operation, in order to adjust its length and the size of its anastomotic part to proper levels. Such a cutting, however, sometimes finds difficulty in performing anastomotic operation, in addition to a possible danger of infection at the operation site, because the cutting frequently causes fraying and production of pieces of thread at the cut site of the artificial blood vessel which has a woven structure. These risks can be reduced by applying the water-impermeable layer.

On the other hand, the water-permeable second layer may be in a form of porous structure to give it sufficient water-permeability and composed of either a single non-absorbable material or a complex of a non-absorbable and an absorbable materials.

The non-absorbable material may includes polyesters, polyurethanes, polyethylenes, polypropylenes, fluoroplastics, and Teflon.

The porous structure may be any water-permeable porous structure, such as a fabric woven or knitted of thread, a fabric in a form of a plaited cord and a complex structure of these fabrics.

As described above, the prosthesis for living organ to be implanted in a body in accordance with the present invention is a laminate of a substantially water-impermeable first layer and water-permeable second layer. It is necessary also to laminate and fix these layers in the case of the application of the prosthesis to a sheet of patch or a tubular artificial blood vessel. The following shows typical examples of the lamination process.
(1) A silicone tube into which a Teflon rod is previously inserted is covered with a film comprising an absorbable material and then inserted into an artificial blood vessel. Thereafter, the film of the absorbable material is coated on the inner surface of the vessel comprising a porous non-absorbable layer using a good solvent for the absorbable material (for example, chloroform, acetone and the like when the absorbable material is a random copolymer of polyglycolate and polylactate).
(2) A tube comprising an absorbable material is enclosed with a liquid which does not dissolve the absorbable material (for example, Freon and the like when the absorbable material is a random copolymer of polyglycolate and polylactate) and then inserted into an artificial blood vessel. Thereafter, the tube of the absorbable material is attached to the inner surface of the vessel comprising a porous non-absorbable layer in the same manner as in the case (1).
(3) A water-impermeable tube is prepared by adhering an absorbable material to a porous tube comprising a non-absorbable material by means of dipping and the like and then the water-impermeable tube is enveloped with a porous tube comprising a non-absorbable material.

An implantation experiment has revealed that the adhesion ability of thrombi formed inside of an artificial blood vessel to the inner surface of the vessel was weak when the surface was smooth, indicating a possible danger of causing obstruction in peripheral blood vessels by the thrombi separated from the inner surface of the artificial blood vessel by the flow of blood. Such a separation of thrombi by the flow of blood can be prevented by making the blood-contacting surface into a rough form such as a porous structure. For this purpose, a surface layer of the inner side may preferably be formed into a porous structure by, for example, (1) partly dissolving surface area of the absorbable layer by dipping the layer into a good solvent for the absorbable material or (2) firstly coating inside of a tube with a solution of an absorbable material and then removing the solvent in the solution before the solvent volatilizes by using a proper solution which does not dissolve the absorbable material. When an artificial blood vessel was prepared by adhering a film of a copolymer of polyhydroxybutylate and polyhydroxyvalerate to the blood-contacting side of the vessel and making the surface of the blood-contacting side of the adhered film into a form of porous structure by means of the process described just above, it was found that the artificial blood vessel thus prepared was water-impermeable, the surface of the blood-contacting side was in a form of porous structure and the flexibility of the vessel was improved by this treatment.

In the case of a small diameter artificial blood vessel (inside diameter, 5 mm or less), anti-thrombus treatment may preferably be performed in some way, because the frequency of causing obstruction is high in such a case due to its higher thrombus-forming effect compared to a large diameter artificial blood vessel. Fixation of an anti-thrombotic agent, such as heparin, in a sustained release form may be effective for this purpose. Mixing of heparin and an absorbable material may easily be achieved. For example, a tube of a copolymer of polylactate and polyglycolate containing heparin can be prepared by dissolving a complex of benzalkonium chloride and heparin and a copolymer of polylactate and polyglycolate into chloroform, casting the solution on the surface of a silicone tube and then air-drying the casted surface. An anti-thrombotic artificial blood vessel having a water-impermeable layer on its inner wall can be prepared by adhering this tube on the inner surface of an artificial blood vessel fabricated from a fabric woven of a polyester fiber material in the same manner as described above.

### EXAMPLES

Examples of the present invention are given below by way of illustration, and not by way of limitation.

### Example 1

An artificial blood vessel of the present invention having a water-impermeable layer as its inner layer was prepared as follows. A silicone tube into which a Teflon (a trademark) rod was previously inserted was covered with a film (100 µm in thickness) comprising a random copolymer of polyhydroxybutylate and polyhydroxyvalerate (mixing ratio, 70:30) and then inserted into a commercially available artificial blood vessel (inside diameter, 8 mm) fabricated from a fabric woven of a polyester fiber material. Thereafter, the inserted film was coated on the inner surface of the polyester vessel using a good solvent for the random copolymer.

The artificial blood vessel of the present invention thus prepared (5.5 cm in length) was implanted in the thoracic aorta of a crossbred adult dog (body weight, 10 to 15 kg) and the effect of the vessel was observed periodically. As the result, it was confirmed that bleeding at the time of the implantation operation was prevented perfectly and penetration of surrounding tissues into the vessel was enhanced by the function of the water-impermeable layer for 1 to 2 weeks after the implantation, followed by a satisfactory healing.

### Example 2

An artificial blood vessel of the present invention having a water-impermeable layer as its inner layer was prepared using a copolymer of polyhydroxybutylate and polyhydroxyvalerate in basically the same manner as described in Example 1. The artificial blood vessel prepared in this case was rough to the touch and low in its flexibility because the layer of the absorbable material itself was hard. An examination was performed in order to find a method to soften the absorbable layer. As the result, it was found that the absorbable layer was able to be softened by mixing the absorbable material with a phospholipid, a fatty acid and the like, or by coating a thin layer of the absorbable material on a sponge of collagen, fibrin and the like.

### Comparative Example 1

Internal surface of a commercially available artificial blood vessel (inside diameter, 4 mm; coefficient of water permeability, 2400 ml/cm²) fabricated from a fabric woven of a polyester fiber material was coated with a layer of fibrous collagen and the coated layer was included with arginine and treated with glutaraldehyde. A heparinized anti-thrombotic artificial blood vessel was prepared by soaking this glutaraldehyde-treated vessel into 1% sodium heparinate solution (pH 6.0) for 12 hours at room temperature.

When a physiological saline was injected under pressure in the cavity of the heparinized anti-thrombotic artificial blood vessel thus prepared, the physiological saline was leaked out through the vessel wall, showing that this vessel had water-permeability.

The heparinized anti-thrombotic artificial blood vessel thus prepared (4 mm in inside diameter and 6 cm in length) was implanted in the femoral aorta of a crossbred adult dog (body weight, 15 to 20 kg) and the effect of the vessel was observed periodically.

The implantation was able to be performed in similar manner to the process for the implantation of commonly used artificial blood vessels, by neutralizing heparin at the sutured part with drops of a solution of protamic sulfate in order to stanch bleeding from the sutured part.

The external surface of the artificial blood vessel wall was reddish just after the implantation, showing soaking of blood components but with no bleeding. The internal surface of the artificial blood vessel wall just after the implantation was also reddish, but with no evidence of the adhesion of thrombi.

The artificial blood vessel showed its performance without causing obstruction of the tube one week after the implantation, but with a leakage of liquid components of blood from the artificial blood vessel wall and the accumulation of aseptic exudation on the peripheral area of the vessel. According to a light microscopic observation, the penetration of fibroblasts, capillary blood vessels and the like into the vessel wall was not found, probably due to the accumulation of exudation. The artificial blood vessel still showed its performance without causing obstruction of the tube two week after the implantation, but with the accumulation of similar exudation on the peripheral area of the vessel. The exudation, however, showed no increasing tendency probably due to its balanced leaking and absorption.

According to an optical microscopic observation, the penetration of fibroblasts and capillary blood vessels into the vessel wall was not found even two weeks after the implantation. After three weeks of the implantation, the artificial blood vessel was found in an obstructed form.

### Comparative Example 2

A commercially available artificial blood vessel (inside diameter, 4 mm; length, 6 cm; and coefficient of water permeability, 2400 ml/cm²) fabricated from a fabric woven of a polyester fiber material was implanted in the femoral aorta of a crossbred adult dog (body weight, 15 to 20 kg) and the effect of the vessel was observed periodically.

Pre-clotting treatment was required at the time of its implantation because of significant bleeding from the artificial blood vessel wall. This artificial blood vessel was found in an obstructed form 24 hours after its implantation.

Thus, it is apparent that there have been provided, in accordance with the present invention, a prosthesis for living organ adapted for such use as an artificial blood vessel and a patch for blood vessel. This invention also provides an artificial blood vessel prepared from such prosthesis and a process for preparing thereof.

A prosthesis for living organ to be implanted in a body at such site is provided wherein the surface of a water-impermeable layer of the prosthesis contacts with blood. The water-impermeable layer comprises an absorbable material which is capable of being gradually absorbed and removed from the prosthesis within the survival period of the patient, the prosthesis consequently becoming water-permeable.

The prosthesis of the present invention can be applied to porous artificial blood vessels and patches for blood vessels, which can be used for a long time due to the ability of the absorbable water-impermeable layer to reduce the risk of causing infection by pieces of thread or fraying at a cut site of the prosthesis at the time of implantation operation, to prevent bleeding from the wall of an artificial blood vessel after its implantation, and to prevent obstruction of an artificial blood vessel through the formation of stable pseudo-endothelium.

Adhesion of thrombi onto the surface of the blood-contacting surface of the absorbable water-impermeable layer of the present invention in an artificial blood vessel can be improved by making the surface into a form of porous structure. Thrombus-originated obstruction of an artificial blood vessel, which occurs frequently when diameter of the vessel is small, can be prevented by mixing the absorbable material of the present invention with an anti-thrombotic agent, such as heparin. In addition, commonly used complex pre-clotting treatment to prevent bleeding from inside of an artificial blood vessel is not required, because the prosthesis of the present invention is water-impermeable at the time of implantation operation.

## Claims

1. A prosthesis for living organ to be implanted in a body at such site wherein one major surface of the prosthesis contacts with blood, comprising a laminate of
a first layer (1) of an absorbable material which is capable of being gradually absorbed through decomposition or metabolism of the material such that the absorbable material is removed from the prosthesis within the survival period of the patient,
said first layer (1) being provided on the blood-contacting side of the prosthesis, and
a water-permeable second layer (2) primarily comprising a non-absorbable material for supporting the prosthesis and imparting strength thereto, which material is not absorbed in the body within the lifetime, characterized in that said first layer (1) is substantially water-impermeable, and said prosthesis becomes water-permeable after removal of said absorbable material.

2. The prosthesis of claim 1 wherein said non-absorbable material is in a form of a porous structure.

3. The prosthesis of claim 1 wherein said absorbable material contains an anti-thrombotic agent.

4. The prosthesis of claim 1 wherein said absorbable material is an absorbable polyester selected from the group consisting essentially of polylactate, polyglycolate, polyhydroxybutylate, polyhydroxyvalerate, poly-ε-caprolactone ester, polyethylene adipate and their copolymers.

5. The prosthesis of claim 4 wherein said absorbable polyester contains at least a member selected from the group consisting essentially of polyamino acids, phospholipids or fatty acids.

6. The prosthesis of claim 1 wherein said non-absorbable material is selected from the group consisting essentially of polyesters, polyurethanes, polyethylenes, polypropylenes, fluoroplastics, and polytetrafluoroethylene.

7. The prosthesis according to claim 1 wherein said absorbable material is impregnated in said porous structure.

8. An artificial blood vessel fabricated from the prosthesis of claim 1.

9. A process of preparing a prosthesis according to claim 1 for living organ to be implanted in a body, wherein a water-impermeable first layer (1) comprising an absorbable material is formed on a water-permeable second layer (2) primarily containing a non-absorbable material by inserting a cylindrical form of film comprising said absorbable material into at least a portion of cavity of an artificial blood vessel prepared from said second layer (2).

## Patentansprüche

1. Prothese für ein lebendes Organ, die an einer Stelle, an der eine Hauptfläche der Prothese mit Blut in Berührung gelangt, in einen Körper implantiert werden soll, umfassend ein Verbundgebilde aus
einer ersten Schicht (1) aus einem absorbierbaren Material mit der Fähigkeit, nach und nach durch Zersetzung oder Verstoffwechselung derart absorbiert zu werden, daß es während der Überlebensdauer des Patienten aus der Prothese entfernt wird, wobei die erste Schicht (1) auf der Blutkontaktseite der Prothese vorgesehen ist, und
einer wasserdurchlässigen zweiten Schicht (2), die vornehmlich aus einem innerhalb des Lebenszeitraums nicht in den Körper absorbierten, nicht-absorbierbaren Material zum Stützen oder Tragen der Prothese besteht und ihr Festigkeit verleiht,
dadurch gekennzeichnet, daß die erste Schicht (1) praktisch wasserundurchlässig ist und die Prothese nach Entfernung des absorbierbaren Materials wasserdurchlässig wird.

2. Prothese nach Anspruch 1, wobei das nicht-absorbierbare Material in einer porösen Strukturform vorliegt.

3. Prothese nach Anspruch 1, wobei das absorbierbare Material ein gerinnungshemmendes Mittel enthält.

4. Prothese nach Anspruch 1, wobei das absorbierbare Material aus einem absorbierbaren Polyester, der im wesentlichen aus der Gruppe Polylactat, Polyglycolat, Polyhydroxybutylat, Polyhydroxyvalerat, Poly-ε-caprolactonester, Polyethylenadipat und deren Copolymeren ausgewählt ist, besteht.

5. Prothese nach Anspruch 4, wobei der absorbierbare Polyester mindestens eine Komponente, die im wesentlichen aus der Gruppe Polyaminosäuren, Phospholipiden oder Fettsäuren besteht, enthält.

6. Prothese nach Anspruch 1, wobei das nicht-absorbierbare Material aus der Gruppe, die im wesentlichen aus Polyestern, Polyurethanen, Polyethylenen, Polypropylenen, Fluorkunststoffen und Polytetrafluorethylen besteht, ausgewählt ist.

7. Prothese nach Anspruch 1, wobei das absorbierbare Material in die poröse Struktur imprägniert ist.

8. Künstliches Blutgefäß, hergestellt aus der Prothese nach Anspruch 1.

9. Verfahren zur Herstellung einer Prothese nach Anspruch 1 für ein in einen Körper zu implantierendes lebendes Organ, wobei eine ein absorbierbares Material umfassende, wasserundurchlässige erste Schicht (1) auf einer hauptsächlich ein nicht-absorbierbares Material enthaltenden, wasserdurchlässigen zweiten Schicht (2) durch Einfügen eines das absorbierbare Material umfassenden zylinderförmigen Films in mindestens einen Teil einer Ansnehmung eines aus der zweiten Schicht (2) hergestellten künstlichen Blutgefäßes ausgebildet wird.

## Revendications

1. Prothèse d'organe vivant à implanter dans un corps en un endroit où la plus grande partie de la surface de la prothèse est en contact avec le sang, comprenant un stratifié fait de:
- une première couche (1) en matériau absorbable, capable d'être progressivement absorbée par décomposition ou métabolisme du matériau, de sorte que le matériau absorbable est éliminé de la prothèse pendant la période de survie du patient,
ladite première couche (1) étant placée sur le côté de la prothèse en contact avec le sang, et
- une seconde couche (2) perméable à l'eau, principalement composée d'un matériau non absorbable, pour supporter la prothèse et lui donner une certaine résistance mécanique, matériau qui n'est pas absorbé dans le corps pendant la vie,
caractérisée en ce que ladite première couche (1) est essentiellement imperméable à l'eau et ladite prothèse devient perméable à l'eau après élimination dudit matériau absorbable.

2. Prothèse selon la revendication 1, dans laquelle ledit matériau non absorbable a une forme de structure poreuse.

3. Prothèse selon la revendication 1, dans laquelle ledit matériau absorbable contient un agent anti-thrombotique.

4. Prothèse selon la revendication 1, dans laquelle ledit matériau absorbable est un polyester absorbable choisi dans le groupe formé essentiellement par les polylactates, les polyglycolates, les poly(hydroxybutylate), les poly(hydroxyvalérate), les polyester-ε-caprolactones, les poly(adipate d'éthylène) et leurs copolymères.

5. Prothèse selon la revendication 4, dans laquelle ledit polyester absorbable contient au moins un élément choisi dans le groupe formé essentiellement des acides polyaminés, des phospholipides et des acides gras.

6. Prothèse selon la revendication 1, dans laquelle ledit matériau non absorbable est choisi dans le groupe formé essentiellement des polyesters, des polyuréthanes, des polyéthylènes, des polypropylènes, des plastiques fluorés et du polytétrafluoréthylène.

7. Prothèse selon la revendication 1, dans laquelle ledit matriau absorbable est imprégné dans ladite structure poreuse.

8. Vaisseau sanguin artificiel fabriqué à partir de la prothèse de la revendication 1.

9. Procédé de préparation d'une prothèse d'organe vivant conforme à la revendication 1, à implanter dans un corps, dans lequel une première couche (1) imperméable à l'eau, comprenant un matériau absorbable, est formée sur une seconde couche (2) perméable à l'eau, contenant principalement un matériau non absorbable, par introduction d'une forme cylindrique de pellicule comprenant ledit matériau absorbable dans une partie au moins de la cavité d'un vaisseau sanguin artificiel préparé à partir de ladite seconde couche (2).
